# EUROPEAN PATENT SPECIFICATION

(11) **EP 1 804 744 B1**
(45) Date of publication and mention of the grant of the patent: **27.02.2013**
(21) Application number: 05790885.7
(22) Date of filing: 26.09.2005
(51) Int. Cl.: A61F 5/443

(54) **REINFORCED PADS FOR OSTOMY BAGS**
VERSTÄRKTE EINLAGEN FÜR OSTOMIE-BEUTEL
BOURRELETS RENFORCÉS POUR SACS STOMIQUES

(30) Priority: 24.09.2004 GB 0421289
(43) Date of publication of application: 11.07.2007
(73) Proprietor: WELLAND MEDICAL LIMITED, Crawley, West Sussex RH10 2TU (GB)
(72) Inventor: SMITH, Rory, Welland Medical Limited, Crawley West Sussex RH10 2TU (GB); MILLS, Barrie, Welland Medical Limited, Crawley West Sussex RH10 2TU (GB)
(74) Representative: Fry, David John
(86) International application number: PCT/GB2005/003706
(87) International publication number: WO 2006/032924

(56) References cited:
- EP-A- 0 415 282
- EP-A- 1 378 219
- GB-A- 2 347 863
- US-A- 5 718 696
- US-A1- 2003 060 786

## Description

Ostomy bags, such as urostomy, ileostomy and colostomy bags, are used to collect bodily waste draining from a stomal opening in the patient's body wall. Ostomy bags may be secured to the patient by means of a belt or strap, and/or adhesive, but more usually they are affixed to the patient by means of an adhesive flange which surrounds the stomal orifice.

For many ostomy bag wearers, the stomal opening is sited in a recess in the body wall. This may be a consequence of patient weight gain after intestinal surgery or the particular surgery performed. In such cases it is preferable to use an ostomy bag in conjunction with a deformable pad which has a body contacting surface which is generally convex in shape in order that the skin surrounding the opening of the stoma is contacted and adhered to the adhesive surface of the appliance. Such ostomy bag appliances incorporating a deformable pad are commonly referred to as "convexity" appliances.

Conventionally, "convexity" appliances include a reinforcing member which is, for example formed of an injection-moulded plastics material to reinforce and stiffen the region around the stomal opening to form and maintain the convex shape.

Such appliances are generally expensive to manufacture and have led to patient complaints particularly resulting from the pressure required to fix the appliance in place. Complaints include *inter alia,* skin damage, such as ulceration and bruising as well as general discomfort. Not only are such appliances difficult to attach to the body wall so that the stomal orifice is aligned with the opening in the ostomy bag, but they do not accommodate the full range of body movements, such as reaching and bending, and therefore may lead to a loss of adhesion between the deformable pad and the body or further physical discomfort. Furthermore, the rigid edge of the moulding in the region of the orifice may damage the stoma. The shape and stiffness of the moulding cannot be easily altered between patients and so such stiffening elements cannot be efficiently adapted to better suit the needs of individual patient's.

The shape of the stoma is likely to vary between patients but altering the shape of the injection moulded body attachment is timely and expensive and as such is impractical on a patient to patient basis, as alteration of the relevant tooling is required.

EP 0415282 relates to an ostomy device with convex adhesive face plate and protective shield and a method for fabricating the same. The document discloses a plastic annular dish which has a convex exterior surface upon which is situated an adhesive wafer. A removeable protective shield is formed to have a substantially wrinkle-free surface with a contour identical to that of the convex surface. The dish and wafer are put in a vacuum chamber, below a planar plastic sheet. The top of the chamber is heated. The space above the sheet is evacuated to draw the sheet against the heated surface. The remaining portion of the chamber is then evacuated to eliminate air between the wafer and dish. Lastly, the top of the chamber is vented such that the heated sheet is pressed over the wafer and dish.

The present Invention seeks to provide a convexity appliance which is relatively easy to manufacture and allows the rigidity and shape of the deformable pad to be controlled. A hot melt adhesive is employed to secure components of the device to each other.

Furthermore, the present invention seeks to provide an appliance which overcomes the problems associated with the earlier appliances by improving the rigidity of the deformable pad by the application of an adhesive.

By "adhesive" it is meant a hot melt adhesive which is applied in molten form and solidifies on cooling.

According to claim 1 the invention provides a deformable pad for removably securing an ostomy bag to the skin of a patient; the pad having an opening for receiving stomal waste, the opening being surrounded by a convex formation on a bodyside face of the pad and the convex formation being reinforced by a hot melt adhesive applied on one surface of the pad.

Independent claim 14 refers to method of producing such a pad.

Preferably, the surface is that opposite the bodyside surface.

Preferably, the opening of the pad may have a convex lip.

The seal of the pad to a user's skin may be improved by having a convex lip at the opening.

In one embodiment, the convex formation, surrounding the opening and reinforced by a hot melt adhesive, may be inside the bag.

This embodiment allows the reinforced convex formation to be less conspicuous and the deformable pad is more comfortable for a user.

By avoiding the need for separately manufactured stiffening elements the shape and stiffness of any one pad may be easily and efficiently adapted. The deformable pad may be formed of any shape suitable for use in a stomal appliance context. The annulus of the pad may be generally circular or alternatively may be generally oval shape, for example.

Adhesive may be applied in a specific design or shape depending on specific requirements. For example, the adhesive may be applied in an oval or figure of eight shape. Such a shape may offer increased comfort and usability to a patient with a double stoma, for example.

The hot melt adhesive may preferably be applied through a nozzle attached to an applicator.

More preferably, the applicator nozzle rotates in a spiral to avoid any discontinuities in the application of the hot melt adhesive.

The rigidity of a pad may be controlled by altering the grade or amount of hot melt adhesive that is applied.

The hot melt adhesive may be applied to any shape of deformable pad provided that there is some kind of convex formation.

The hot melt adhesive may be applied in generally uniform thickness. Alternatively, the hot melt adhesive may vary in thickness depending on the level of reinforcement required at particular locations within the convex formation.

Preferably, the applicator nozzle is computer operated such that the direction, velocity and amount of hot melt adhesive may be automated.

The pressure required to be applied in the region of the opening may be reduced (or increased) by changing computer operation of the applicator used to apply the hot melt adhesive.

The application of hot melt adhesive may preferably be controlled remotely.

In a further aspect, the invention provides a method for producing a reinforced deformable pad, the method comprising the steps of: -
a) providing a layer of thin film, or a precursor thereof, and a layer of adhesive material;
b) shaping the layers of material into a convex pad; and,
c) bonding the shaped layers together,
d) attaching the bonded convex pad to an ostomy bag leaving a convex area exposed, and
e) applying a hot melt adhesive to the convex area and allowing the adhesive to harden.

The term "film" is used herein to denote a thin layer of material comprising a coherent layer or fused mesh. The term "a precursor thereof" is used herein to denote a material which may be used to form a film, for example a woven or non-woven material which on heating forms a film.

Preferably the layer of thin film is made of a plastics material.

The bodyside surface of the deformable pad may be covered or coverable with a release liner which may be made of a moulded plastics film. The release liner preferably conforms to the shape of the bodyside surface of the pad. The liner may be pre-formed e.g. by vacuum forming, but more preferably it is vacuum formed as a laminate together with the adhesive layer. The liner may be made from styrene, polyethylene, or PVC, e.g. high density or medium density. The liner may be coated with a silicone on one or both sides.

The layers may be shaped and bonded together simultaneously, for example, by thermo-forming or vacuum-forming.

In one embodiment, the layers are shaped and bonded with a release liner. The release liner conforms to the shape of the pad and protects the adhesive surface of the pad. The release liner may be pre-formed.

More preferably however, the film (or precursor thereof) and adhesive material and optionally the release liner may be laminated together before shaping.

In one embodiment of the present invention a cooling device is used to harden the hot melt adhesive.

Preferably the cooling device comprises a hollow contoured brass block coated with a silicone membrane.

Preferably a coolant is passed through the brass block.

In one embodiment the brass block is corrugated.

The brass block may preferably incorporate a stamp to produce an identifiable mark on the hot melt adhesive which is visible when the adhesive hardens.

The invention will now be described by way of example only by reference to the accompanying, diagrammatic drawings in which:-
Figure 1 is a partial side elevation of a deformable pad and ostomy bag according to the invention, and
Figure 2 is a side view of a cooling device used in the process of constructing a deformable pad according to the invention.

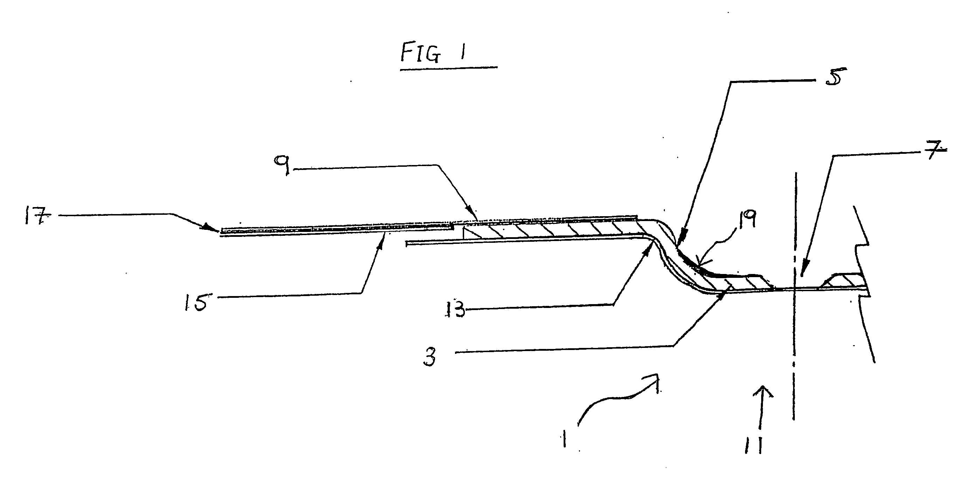

Referring now to Figure 1, a deformable pad 1 according to the invention comprises an adhesive pad 3, having a convex formation 5. The pad 3 is formed of a thin plastics layer such as non-woven polyethylene, bonded to a bagside surface of a layer of hydrocolloid adhesive material such as a polyisobutylene containing modified polysaccharides. The pad 3 is provided with a generally central aperture 7 through which a stoma of a patient may protrude. The central aperture is surrounded by a convex lip. Overlying the pad 3 is a backing layer 9 formed of polyethylene non-woven material.

The bodyside surface 11 of the adhesive material is temporarily covered and protected by the use of a release liner 13 made of a moulded plastics film (e.g. polyethylene terephthalate (PET)) which may be removed immediately prior to use of the pad 3.

In this embodiment, the deformable pad 1 is produced using vacuum forming. A layer of thin plastics film, in this embodiment, the layer of non-woven polyethylene 9, is placed adjacent to the layer of hydrocolloid adhesive material 3. The hydrocolloid adhesive layer 3 is placed on the release liner 13 which is made of pre-formed, silicone-coated, PET. By way of example, the release liner 13 may be from about 80 microns to 180 microns thick, the hydrocolloid adhesive layer 3 may be from 0.9 mm to 1.2 mm thick, and the non-woven layer backing layer 9 may be approximately 0.2 to 0.6 mm thick, more preferably 0.3 mm thick.

The three layers are initially laminated together using an extrusion and laminating line and are then cut to the desired shape. Typically the blank thus formed is of a generally circular shape, but with one or more part-circular tab portions being provided around the periphery of the circle to facilitate fixing and removal of the adhesive pad to and from the patient. The release liner 13 typically is formed with a hole at the centre thereof, although it need not be.

The planar laminate blank is then placed on a vacuum forming machine, the moulding tool having a convex form. A means of heating, such as a radiant heater, is positioned above the laminating machine in order to soften the laminate, and the laminate is then subjected to vacuum forming for a period of, for example, five to seven seconds.

In an alternative embodiment the deformable pad 3 may be produced using a heat and pressure die.

The deformable pad 1 may be formed of any shape suitable for use in a stomal appliance context. The annulus of the pad 3 may be generally circular or alternatively may be generally oval shape, for example.

A convexly formed laminate 1 removed from the mould may be secured to an ostomy bag 15 in accordance with methods known per se. The surface of the backing layer 9 which is opposite to the bodyside surface 11 of the pad is bonded to the ostomy bag 15, by means of adhesive 17. The ostomy bag 15 may also be attached by welding.

The deformable pad 1 maintains its convex formation 5 on attachment to the bag 15 leaving a "dish" portion exposed on the opposite side of the pad to the bodyside surface 11.

The deformable pad 1 is reinforced by application of a layer of hot melt adhesive 19 extending at least partially around the convex formation 5 opposite to the bodyside surface 11 of the pad 1.

The adhesive 19 is applied using an applicator nozzle of an applicator which is programmed to move towards, and in relation to, the dish portion to apply hot melt adhesive 19 in a rotating spiral movement.

The pressure required to be applied in the region of the opening may be reduced (or increased) by changing the programmed plot of the applicator.

The hot melt adhesive 19 can be applied in a specific design or shape depending on specific requirements. For example, the adhesive 19 can be applied in an oval or figure of eight shape.

The rigidity of the pad 3 may be controlled by altering the grade or amount of hot melt adhesive 19 that is applied.

The adhesive 19 hardens to reinforce and strengthen the convex formation 5.

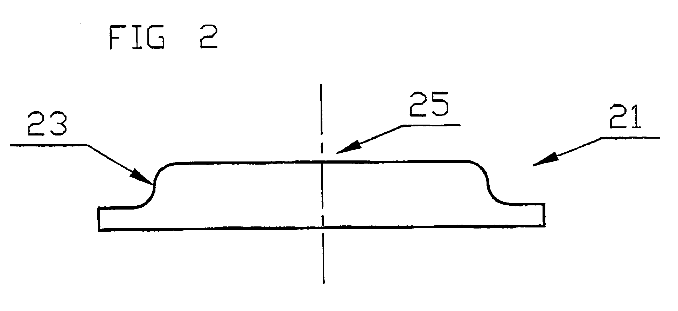

Figure 2 illustrates a cooling device 21 used to ensure that heat is quickly removed from the hot melt adhesive 19. Preferably the adhesive is hardened in six seconds or less. A hollow brass block 23 is contoured to the shape of the convex formation of the deformable pad 3 and covered with a silicone membrane 25. A coolant is passed through the brass block 23 such that when the cooling device 21 is brought into contact with the hot melt adhesive 19, the adhesive 19 rapidly cools and hardens.

In use, the deformable pad 1 is affixed to the skin of the patient about the stomal opening, the hydrocolloid adhesive 3 serving to adhere the convex formation 5 and the remainder of the pad 1 to the skin of the patient.

It will be understood that the foregoing is merely exemplary of an embodiment of the invention and that modification may be made without departing from the true scope of the invention.

## Claims

1. A deformable pad (1) for removably securing an ostomy bag (15) to the skin of a patient; the pad having an opening (7) for receiving stomal waste, the opening (7) being surrounded by a convex formation (5) on a bodyside face of the pad (11) **characterised by** a hot melt adhesive(19) application a thickness only on one surface of the convex formation so that the convex formation is reinforced when the adhesive is hardened.

2. A deformable pad (1) according to claim 1 wherein the hot melt adhesive (19) is applied to a surface that is opposite to the bodyside surface.

3. A deformable pad (1) according to any preceding claim wherein the opening of the pad (7) comprises a convex lip.

4. A deformable pad (1) according to any preceding claim wherein the convex formation, (5) surrounding the opening (7) and reinforced by a hot melt adhesive (19), is inside the bag (15).

5. A deformable pad (1) according to any preceding claim wherein the opening of the pad (7) is circular.

6. A deformable pad (1) according to any of claims 1 to 5 wherein the opening of the pad (7) is oval.

7. A deformable pad (1) according to any preceding claim wherein the hot melt adhesive (19) Is applied in an oval shape.

8. A deformable pad (1) according to any of claims 1 to 6 wherein the hot melt adhesive (19) is applied in a figure of eight shape.

9. A deformable pad (1) according to any preceding claim wherein the bodyside face of the deformable pad (11) is covered or coverable with a release liner (13).

10. A deformable pad (1) according to claim 9 wherein the release liner (13) is a moulded plastics film.

11. A deformable pad (1) according to any of claim 9 or claim 10 wherein the release liner (13) conforms to the shape of the bodyside surface of the pad (11).

12. A deformable pad (1) according to any preceding claim wherein the hot melt adhesive (19) is of uniform thickness.

13. A deformable pad (1) according to any of claims 1 to 11 wherein the hot melt adhesive (19) is of variable thickness.

14. A method of producing a reinforced deformable pad (1) according to claim 1 comprising the steps of: -
providing a layer of thin film, (9) or a precursor thereof, and a layer of adhesive material; (3)
shaping the layers of material (3, 9) into a convex pad;
bonding the shaped layers (3, 9) together,
attaching the bonded convex pad to an ostomy bag (15) leaving a convex formation (5) exposed, **characterised by**
applying a hot melt adhesive in a thickness only on one surface of the convex formation(5), so that said convex formation is reinforced cohen the adhesive (19) hardens.

15. A method of producing a reinforced deformable pad (1) according to claims 14 wherein the thin film (9) is a coherent layer or a fused mesh.

16. A method of producing a reinforced deformable pad (1) according to claim 14 wherein the precursor of the thin film (9) is a woven or non-woven material which on heating forms a thin film (9).

17. A method of producing a reinforced deformable pad (1) according to any of claims 14 to 16 wherein the thin film (9) is a plastics material.

18. A method of producing a reinforced deformable pad (1) according to claim 17 wherein the layer of thin film (9) is any one of styrene, polyethylene, high density PVC or medium density PVC.

19. A method of producing a reinforced deformable pad (1) according to any of claims 14 to 18 wherein the thin film (9) is coated with a silicone on one or both sides.

20. A method of producing a reinforced deformable pad (1) according to any of claims 14 to 19 wherein the convex pad is pre-formed.

21. A method of producing a reinforced deformable pad (1) according to claim 20 wherein the convex pad is vacuum formed.

22. A method of producing a reinforced deformable pad (1) according to claim 21 wherein the convex pad is vacuum formed as a laminate together with the layer of adhesive material (3).

23. A method of producing a reinforced deformable pad (1) according to any of claims 14 to 22 wherein the thin film layer (9) and adhesive material (3) are shaped and bonded together simultaneously.

24. A method of producing a reinforced deformable pad (1) according to any of claims 22 or 23 wherein the thin film layer (9) and adhesive material (3) are shaped and bonded together by either thermo-forming or vacuum-forming.

25. A method of producing a reinforced deformable pad (1) according to any of claims 14 to 24 further comprising providing a release liner (13).

26. A method of producing a reinforced deformable pad (1) according to claim 25 wherein the release liner (13) conforms to the shape of the convex pad and/or protects the adhesive surface of the pad.

27. A method of producing a reinforced deformable pad (1) according to any of claims 25 or 26 wherein the release liner (13) may be pre-formed.

28. A method of producing a reinforced deformable pad (1) according to any of claims 25 to 27 wherein the thin film (9), adhesive material (3) and release liner (13) are laminated together before shaping.

29. A method of producing a reinforced deformable pad (1) according to any of claims 14 to 28 wherein a cooling device (21) is used to harden the hot melt adhesive (19).

30. A method of producing a reinforced deformable pad (1) according to claim 29 wherein the cooling device (21) comprises a hollow contoured brass block (23) coated with a silicone membrane.

31. A method of producing a reinforced deformable pad (1) according to claim 30 wherein a coolant is passed through the brass block (23).

32. A method of producing a reinforced deformable pad (1) according to any of claims 30 or 31 wherein the brass block (23) is corrugated.

33. A method of producing a reinforced deformable pad (1) according to any of claims 30 to 32 wherein the brass block (23) incorporates a stamp to produce a mark on the hot melt adhesive (19) which is visible when the adhesive (19) hardens.

34. A method for production of the deformable pad (1) according to claim 14 wherein the hot melt adhesive (19) is applied through a nozzle.

35. A method according to claim 34 wherein the nozzle is attached to an applicator.

36. A method according to claim 34 or claim 35 wherein the nozzle is rotated.

37. A method according to claim 36 wherein the nozzle is rotated in a spiral.

38. A method according to any of claims 34 to 37 wherein the nozzle is computer operated.

39. A method according to any of claims 35 to 38 wherein any of the direction, velocity or volume of hot melt adhesive (19) which is applied is automated.

40. A method according to any of claims 34 to 39 wherein the nozzle is controlled remotely.

## Patentansprüche

1. Eine verformbare Einlage (1) für das entfernbare Befestigen eines Ostomie-Beutels (15) an der Haut eines Patienten; wobei die Einlage eine Öffnung (7) zum Aufnehmen von Stoma-Ausscheidungen hat, wobei die Öffnung (7) umgeben ist von einer konvexen Struktur (5) auf der körperseitigen Fläche der Einlage (11), **dadurch gekennzeichnet, dass** ein durch Wärme aktivierbarer Kleber (19) in einer Stärke nur auf einer Oberfläche der konvexen Struktur aufgebracht ist, so dass die konvexe Struktur verstärkt ist, wenn der Kleber gehärtet ist.

2. Eine verformbare Einlage (1) nach Anspruch 1, wobei der durch Wärme aktivierbare Kleber (19) auf einer Oberfläche aufgebracht ist, die der körperseitigen Oberfläche entgegengesetzt ist.

3. Eine verformbare Einlage (1) nach einem der vorangehenden Ansprüche, wobei die Öffnung der Einlage (7) einen konvexen Rand umfasst.

4. Eine verformbare Einlage (1) nach einem der vorangehenden Ansprüche, wobei die konvexe Struktur, (5) die die Öffnung (7) umgibt und verstärkt ist durch einen durch Wärme aktivierbaren Kleber (19), in dem Beutel (15) gelegen ist.

5. Eine verformbare Einlage (1) nach einem der vorangehenden Ansprüche, wobei die Öffnung der Einlage (7) kreisförmig ist.

6. Eine verformbare Einlage (1) nach einem der Ansprüche 1 bis 5, wobei die Öffnung der Einlage (1) oval ist.

7. Eine verformbare Einlage (1) nach einem der vorangehenden Ansprüche, wobei der durch Wärme aktivierbare Kleber (19) in einer ovalen Form aufgebracht ist.

8. Eine verformbare Einlage (1) nach einem der Ansprüche 1 bis 6, wobei der durch Wärme aktivierbare Kleber (19) in einer Achterform aufgebracht ist.

9. Eine verformbare Einlage (1) nach einem der vorangehenden Ansprüche, wobei die körperseitige Fläche der verformbaren Einlage (1) mit einem Trennpapier (13) bedeckt oder bedeckbar ist.

10. Eine verformbare Einlage (1) nach Anspruch 9, wobei das Trennpapier (13) eine geformte Kunststofffolie ist.

11. Eine verformbare Einlage (1) nach einem der Ansprüche 9 oder 10, wobei das Trennpapier (13) mit der Form der körperseitigen Oberfläche der Einlage (11) übereinstimmt.

12. Eine verformbare Einlage (1) nach einem der vorangehenden Ansprüche, wobei der durch Wärme aktivierbare Kleber (19) von konstanter Stärke ist.

13. Eine verformbare Einlage (1) nach einem der Ansprüche 1 bis 11, wobei der durch Wärme aktivierbare Kleber (19) von variabler Stärke ist.

14. Ein Verfahren zum Herstellen einer verstärkten verformbaren Einlage (1) nach Anspruch 1, umfassend die Schritte von:
Bereitstellen einer Schicht einer dünnen Folie, (9) oder eines Vorprodukts davon, und einer Schicht klebenden Materials; (3)
Formen der Schichten von Material (3, 9) zu einer konvexen Einlage;
Verbinden der geformten Schichten (3, 9) miteinander,
Anfügen der verbundenen konvexen Einlage an einen Ostomie-Beutel (15), wobei eine konvexe Struktur (5) exponiert bleibt, **gekennzeichnet durch**
Aufbringen eines **durch** Wärme aktivierbaren Klebers in einer Stärke nur auf eine Oberfläche der konvexen Struktur (5), so dass besagte konvexe Struktur verstärkt wird, wenn der Kleber (19) härtet.

15. Ein Verfahren zum Herstellen einer verstärkten verformbaren Einlage (1) nach Anspruch 14, wobei die dünne Folie (9) eine kohärente Schicht oder ein verschmolzenes Gewebe ist.

16. Ein Verfahren zum Herstellen einer verstärkten verformbaren Einlage (1) nach Anspruch 14, wobei das Vorprodukt der dünnen Folie (9) ein gewebtes oder nicht-gewebtes Material ist, das bei Erwärmung eine dünne Folie (9) bildet.

17. Ein Verfahren zum Herstellen einer verstärkten verformbaren Einlage (1) nach einem der Ansprüche 14 bis 16, wobei die dünne Folie (9) ein Kunststoffmaterial ist.

18. Ein Verfahren zum Herstellen einer verstärkten verformbaren Einlage (1) nach Anspruch 17, wobei die Schicht der dünnen Folie (9) eines ist von Styren, Polyethylen, PVC hoher Dichte oder PVC mittlerer Dichte.

19. Ein Verfahren zum Herstellen einer verstärkten verformbaren Einlage (1) nach einem der Ansprüche 14 bis 18, wobei die dünne Folie (9) auf einer Seite oder beiden Seiten mit Silikon beschichtet ist.

20. Ein Verfahren zum Herstellen einer verstärkten verformbaren Einlage (1) nach einem der Ansprüche 14 bis 19, wobei die konvexe Einlage vorgeformt ist.

21. Ein Verfahren zum Herstellen einer verstärkten verformbaren Einlage (1) nach Anspruch 20, wobei die konvexe Einlage vakuumgeformt ist.

22. Ein Verfahren zum Herstellen einer verstärkten verformbaren Einlage (1) nach Anspruch 21, wobei die konvexe Einlage vakuumgeformt ist wie ein Laminat zusammen mit der Schicht klebenden Materials.

23. Ein Verfahren zum Herstellen einer verstärkten verformbaren Einlage (1) nach einem der Ansprüche 14 bis 22, wobei die dünne Folienschicht (9) und das klebende Material (3) simultan geformt und miteinander verbunden werden.

24. Ein Verfahren zum Herstellen einer verstärkten verformbaren Einlage (1) nach einem der Ansprüche 22 oder 23, wobei die dünne Folienschicht (9) und das klebende Material (3) geformt und miteinander verbunden werden durch entweder Thermoformen oder Vakuumformen.

25. Ein Verfahren zum Herstellen einer verstärkten verformbaren Einlage (1) nach einem der Ansprüche 14 bis 24 weiterhin umfassend Bereitstellen von Trennpapier (13).

26. Ein Verfahren zum Herstellen einer verstärkten verformbaren Einlage (1) nach Anspruch 25, wobei das Trennpapier (13) mit der Form der konvexen Einlage übereinstimmt und/oder die klebende Oberfläche der Einlage schützt.

27. Ein Verfahren zum Herstellen einer verstärkten verformbaren Einlage (1) nach einem der Ansprüche 25 oder 26, wobei das Trennpapier (13) vorgeformt sein kann.

28. Ein Verfahren zum Herstellen einer verstärkten verformbaren Einlage (1) nach einem der Ansprüche 25 bis 27, wobei die dünne Folie (9), klebendes Material (3) und Trennpapier (13) vor dem Formen zusammenlaminiert sind.

29. Ein Verfahren zum Herstellen einer verstärkten verformbaren Einlage (1) nach einem der Ansprüche 14 bis 28, wobei eine Kühlungsvorrichtung (21) eingesetzt wird zum Härten des durch Wärme aktivierbaren Klebers (19).

30. Ein Verfahren zum Herstellen einer verstärkten verformbaren Einlage (1) nach Anspruch 29, wobei die Kühlungsvorrichtung (21) einen hohlen konturierten Messingblock (23) umfasst, der mit einer Silikonmembran beschichtet ist.

31. Ein Verfahren zum Herstellen einer verstärkten verformbaren Einlage (1) nach Anspruch 30, wobei ein Kühlmittel durch den Messingblock (23) geführt wird.

32. Ein Verfahren zum Herstellen einer verstärkten verformbaren Einlage (1) nach einem der Ansprüche 30 oder 31, wobei der Messingblock (23) gewölbt ist.

33. Ein Verfahren zum Herstellen einer verstärkten verformbaren Einlage (1) nach einem der Ansprüche 30 bis 32, wobei der Messingblock (23) einen Stempel zum Erzeugen einer Markierung auf dem durch Wärme aktivierbaren Kleber (19) enthält, welche beim Härten des Klebers (19) sichtbar wird.

34. Ein Verfahren zum Herstellen einer verstärkten verformbaren Einlage (1) nach Anspruch 14, wobei der durch Wärme aktivierbare Kleber (19) durch eine Düse aufgebracht wird.

35. Ein Verfahren nach Anspruch 34, wobei die Düse an einem Applikator angebracht ist.

36. Ein Verfahren nach Anspruch 34 oder 35, wobei die Düse gedreht wird.

37. Ein Verfahren nach Anspruch 36, wobei die Düse gedreht wird in einer Spirale.

38. Ein Verfahren nach einem der Ansprüche 34 bis 37, wobei die Düse computergesteuert ist.

39. Ein Verfahren nach einem der Ansprüche 35 bis 38, wobei eines von Richtung, Geschwindigkeit oder Volumen von durch Wärme aktivierbarem Kleber (19), der aufgebracht wird, automatisiert ist.

40. Ein Verfahren nach einem der Ansprüche 34 bis 39, wobei die Düse ferngesteuert ist.

## Revendications

1. Bourrelet déformable (1) pour fixer de façon amovible un sac de stomie (15) à la peau d'un patient ; le bourrelet ayant une ouverture (7) pour recevoir les déchets de stomie, l'ouverture (7) étant entourée d'une formation convexe (5) sur une face corporelle du bourrelet (11), **caractérisé par** un adhésif thermofusible (19) appliqué en une épaisseur uniquement sur une surface de la formation convexe de sorte que la formation convexe soit renforcée lorsque l'adhésif est durci.

2. Bourrelet déformable (1) selon la revendication 1, dans lequel l'adhésif thermofusible (19) est appliqué sur une surface qui est opposée à la surface corporelle.

3. Bourrelet déformable (1) selon l'une quelconque des revendications précédentes, dans lequel l'ouverture du bourrelet (7) comprend un bord convexe.

4. Bourrelet déformable (1) selon l'une quelconque des revendications précédentes, dans lequel la formation convexe (5) entourant l'ouverture (7) et renforcée par un adhésif thermofusible (19) est à l'intérieur du sac (15).

5. Bourrelet déformable (1) selon l'une quelconque des revendications précédentes, dans lequel l'ouverture du sac (7) est circulaire.

6. Bourrelet déformable (1) selon l'une quelconque des revendications 1 à 5, dans lequel l'ouverture du bourrelet (7) est ovale.

7. Bourrelet déformable (1) selon l'une quelconque des revendications précédentes, dans lequel l'adhésif thermofusible (19) est appliqué en une forme ovale.

8. Bourrelet déformable (1) selon l'une quelconque des revendications 1 à 6, dans lequel l'adhésif thermofusible (19) est appliqué en une configuration en huit.

9. Bourrelet déformable (1) selon l'une quelconque des revendications précédentes, dans lequel la face corporelle du bourrelet déformable (11) est couverte ou peut être couverte par un revêtement amovible (13).

10. Bourrelet déformable (1) selon la revendication 9, dans lequel le revêtement amovible (13) est un film de plastique moulé.

11. Bourrelet déformable (1) selon l'une quelconque des revendications 9 ou 10, dans lequel le revêtement amovible (13) se conforme à la forme de la surface corporelle du bourrelet (11).

12. Bourrelet déformable (1) selon l'une quelconque des revendications précédentes, dans lequel l'adhésif thermofusible (19) a une épaisseur uniforme.

13. Bourrelet déformable (1) selon l'une quelconque des revendications 1 à 11, dans lequel l'adhésif thermofusible (19) a une épaisseur variable.

14. Procédé de production d'un bourrelet déformable renforcé (1) selon la revendication 1, comprenant les étapes consistant à _{:}
fournir une couche de film fin (9) ou un précurseur de celui-ci et une couche d'un matériau adhésif ; (3)
former les couches de matériau (3, 9) en un bourrelet convexe ;
coller ensemble les couches formées (3, 9),
fixer le bourrelet convexe collé à un sac de stomie (15) laissant une formation convexe (5) exposée, **caractérisé par**
l'application d'un adhésif thermofusible en une épaisseur uniquement sur une surface de la formation convexe (5) de sorte que ladite formation convexe soit renforcée lorsque l'adhésif (19) durcit.

15. Procédé de production d'un sac déformable renforcé (1) selon la revendication 14, dans lequel le film fin (9) est une couche cohérente ou un maillage fusionné.

16. Procédé de production d'un sac déformable renforcé (1) selon la revendication 14, dans lequel le précurseur du film fin (9) est un matériau tissé ou non tissé qui forme lors du chauffage, un film fin (9).

17. Procédé de production d'un sac déformable renforcé (1) selon l'une quelconque des revendications 14 à 16, dans lequel le film fin (9) est une matière plastique.

18. Procédé de production d'un sac déformable renforcé (1) selon la revendication 17, dans lequel la couche de film fin (9) est une couche quelconque de styrène, de polyéthylène, de PVC haute densité ou de PVC moyenne densité.

19. Procédé de production d'un sac déformable renforcé (1) selon l'une quelconque des revendications 14 à 18, dans lequel le film fin (19) est revêtu d'une silicone sur l'un ou les deux côtés.

20. Procédé de production d'un sac déformable renforcé (1) selon l'une quelconque des revendications 14 à 19, dans lequel le bourrelet convexe est préformé.

21. Procédé de production d'un sac déformable renforcé (1) selon la revendication 20, dans lequel le bourrelet convexe est formé sous vide.

22. Procédé de production d'un sac déformable renforcé (1) selon la revendication 21, dans lequel le bourrelet convexe est formé sous vide sous forme de stratifié conjointement avec la couche de matériau adhésif (3).

23. Procédé de production d'un sac déformable renforcé (1) selon l'une quelconque des revendications précédentes 14 à 22, dans lequel la couche de film fin (9) et le matériau adhésif (3) sont formés et liés ensemble simultanément.

24. Procédé de production d'un sac déformable renforcé (1) selon l'une quelconque des revendications 22 ou 23, dans lequel la couche de film fin (9) et le matériau adhésif (3) sont formés et liés ensemble par thermoformage ou formage sous vide.

25. Procédé de production d'un sac déformable renforcé (1) selon l'une quelconque des revendications 14 à 24, comprenant en outre la fourniture d'un revêtement amovible (13).

26. Procédé de production d'un sac déformable renforcé (1) selon la revendication 25, dans lequel le revêtement amovible (13) se conforme à la forme du bourrelet convexe et/ou protège la surface adhésive du bourrelet.

27. Procédé de production d'un sac déformable renforcé (1) selon l'une quelconque des revendications 25 ou 26, dans lequel le revêtement amovible (13) peut être préformé.

28. Procédé de production d'un sac déformable renforcé (1) selon l'une quelconque des revendications 25 à 27, dans lequel le film fin (9), le matériau adhésif (3) et le revêtement amovible (23) sont stratifiés ensemble avant formage.

29. Procédé de production d'un sac déformable renforcé (1) selon l'une quelconque des revendications 14 à 28, dans lequel un dispositif de refroidissement (21) est utilisé pour durcir l'adhésif thermofusible (19).

30. Procédé de production d'un sac déformable renforcé (1) selon la revendication 29, dans lequel le dispositif de refroidissement (21) comprend un bloc en laiton à contour creux (23) revêtu d'une membrane de silicone.

31. Procédé de production d'un sac déformable renforcé (1) selon la revendication 30, dans lequel le refroidissant passe dans le bloc de laiton (23).

32. Procédé de production d'un sac déformable renforcé (1) selon l'une quelconque des revendications 30 ou 31, dans lequel le bloc de laiton (23) est ondulé.

33. Procédé de production d'un sac déformable renforcé (1) selon l'une quelconque des revendications 30 à 32, dans lequel le bloc de laiton (23) comprend une empreinte pour produire une marque sur l'adhésif thermofusible (19) qui est visible lorsque l'adhésif (19) durcit.

34. Procédé de production d'un sac déformable renforcé (1) selon la revendication 14, dans lequel l'adhésif thermofusible (19) est appliqué par une buse.

35. Procédé selon la revendication 34, dans lequel la buse est fixée à un applicateur.

36. Procédé selon la revendication 34 ou 35, dans lequel la buse tourne.

37. Procédé selon la revendication 36, dans lequel la buse tourne en spirale.

38. Procédé selon l'une quelconque des revendications 34 à 37, dans lequel la buse est actionnée par ordinateur.

39. Procédé selon l'une quelconque des revendications 35 à 38, dans lequel l'un quelconque des direction, vélocité ou volume de l'adhésif thermofusible (19) qui est appliqué est automatique.

40. Procédé selon l'une quelconque des revendications 34 à 39, dans lequel la buse est contrôlée à distance.
